# EUROPEAN PATENT SPECIFICATION

(11) **EP 1 946 842 B1**
(45) Date of publication and mention of the grant of the patent: **22.05.2013**
(21) Application number: 07024123.7
(22) Date of filing: 12.12.2007
(51) Int. Cl.: B01L 3/00, C12M 1/22, B01J 19/00

(54) **Biochip kits and methods of testing biological samples using the same**
Biochip-Kits und Verfahren zum Testen von biologischen Proben damit
Kits de biopuces et procédés pour tester des échantillons biologiques les utilisant

(30) Priority: 20.12.2006 KR 20060131211
(43) Date of publication of application: 23.07.2008
(73) Proprietor: Samsung Electronics Co., Ltd., Suwon-si, Gyeonggi-do, 443-742 (KR)
(72) Inventor: Hah, Jung-Hwan, Toyonaka City, Osaka Prefacture 560-0083 (JP); Chi, Sung-Min, Hwaseong-si Gyeonggi-do (KR); Kim, Kyoung-Seon, Suwon-si Gyeonggi-do (KR); Kim, Won-Sun, Suwon-si Gyeonggi-do (KR); Choi, Sang-Jun, Seoul (KR)
(74) Representative: Kuhnen & Wacker

(56) References cited:
- EP-A1- 1 374 996
- WO-A-94/27719
- WO-A-99/39829
- WO-A-2005/066327
- FR-A- 2 861 608

## Description

### 1. Field of the invention

The present invention relates to a method of testing biological samples using molecular detection chip assemblies, in particular to a method of testing a biological sample using a biochip kit including a housing receiving a biochip.

### BACKGROUND OF THE INVENTION

### 2. Description of the Related Art

In recent years, as the genome projects advance, the genome nucleotide sequences for a variety of organisms have been discovered thereby, increasing the interest of using biochips. Further, a variety of biochips are manufactured into kits and applied to test a variety of biological samples. Conventional biochip kits generally include a closed integral housing and a biochip disposed inside the housing. The housing typically forms a reaction space and generally prevents contamination and damage to the biochip.

A typical method of testing a biological sample using a biochip kit is reacting a sample labeled with a fluorescent substance with a probe associated with a biochip by providing the sample to the biochip, and subsequently, then examining light that is emitted from the fluorescent substance by radiating light to the biochip. Generally, a light-detecting lens is disposed relatively close to the biochip to sufficiently concentrate light emitted from a fluorescent substance, with reduced design rules.

However, in conventional biochips, a light-detecting lens may be limited by this approach due, at least in part, to the thickness of the housing. In addition, because light emitted from the housing should be detected outside the housing, at least a part of the housing is formed of a transparent material, such as glass. However, glass can change the optical path and wavelength of light. Therefore, it may be problematic to ensure a sufficient amount of light and detect a reliable wavelength in order to provide meaningful measurements.

A method of testing a biological sample according to the preamble of claim 1 is known from FR 2 861 608 A1 or EP 1 374 996 A1.

### SUMMARY OF THE INVENTION

An object of the present invention is to provide a method of testing a biological sample that can reliably test whether a biological sample is bound to the molecular probes of the biochip included in a biochip kit.

The object is attained by a method of testing a biological sample according to claim 1. Further developments of the invention are specified in the dependent claims.

According to embodiments of the present invention, since a biochip can be exposed to the outside by separating the first and second housings, a detector can approach the exposed biochip with close proximity. Accordingly, since it is possible to not only sufficiently concentrate light, but also detect an accurate wavelength, reliability can be improved in testing procedures.

Further, according to methods of testing a biological sample according to embodiments of the present invention, since it is possible to automatically test several biochip kits in sequence with only an automatic deck and a conveying chuck having a relatively simple structure, test efficiency can be improved.

### BRIEF DESCRIPTION OF THE DRAWINGS

The above and other aspects of the present invention will become more apparent by describing in detail embodiments thereof with reference to the attached drawings in which:
FIG. 1A is a perspective view of a biochip with a first housing and a second housing joined, according to an embodiment of the invention;
FIG. 1B is a cross-sectional view of the biochip of FIG. 1A taken along the line Ib'-Ib;
FIG. 2A is a perspective view of a biochip with first and second housings separated, according to an embodiment of the invention;
FIG. 2B is a cross-sectional view of the biochip of FIG. 2A taken along the line IIb'-IIb;
FIGS. 3 to 8 are cross-sectional views of biochips according to other embodiments of the invention;
FIGS. 9 to 13 are cross-sectional views of biochips included in biochip kits according to some embodiments of the invention;
FIGS. 14, 15A, 16, and 17 are cross-sectional views for illustrating a method of testing a biological sample according to some an embodiments of the invention; and
FIG. 15B is a perspective view illustrating how to grip the first housing of the biochip kits according to some embodiments of the invention.

### DETAILED DESCRIPTION

Advantages and features of the present invention and methods of accomplishing the same may be understood more readily by reference to the following detailed description of embodiments and the accompanying drawings. The present invention may, however, be embodied in many different forms and should not be construed as being limited to the embodiments set forth herein. Rather, these embodiments are provided so that this disclosure will be thorough and complete and will fully convey the concept of the invention to those skilled in the art, and the present invention will only be defined by the appended claims.

The terminology used in the description of the invention herein is for the purpose of describing particular embodiments only and is not intended to be limiting of the invention. As used in the description of the embodiments of the invention and the appended claims, the singular forms "a", "an" and "the" are intended to include the plural forms as well, unless the context clearly indicates otherwise. Also, as used herein, "and/or" refers to and encompasses any and all possible combinations of one or more of the associated listed items.

Unless otherwise defined, all terms, including technical and scientific terms used in this description, have the same meaning as commonly understood by one of ordinary skill in the art to which this invention belongs. All publications, patent applications, patents, and other references mentioned herein are incorporated by reference in their entirety.

It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof.

Moreover, it will be understood that steps comprising the methods provided herein can be performed independently or at least two steps can be combined. Additionally, steps comprising the methods provided herein, when performed independently or combined, can be performed at the same temperature and/or atmospheric pressure or at different temperatures and/or atmospheric pressures without departing from the teachings of the present invention.

In addition, the embodiments of the present invention are described with reference to sectional views and schematic views provided as ideal illustrations of the present invention. As such, the type of illustration may vary depending on the fabrication technique and/or allowable error. Thus, the embodiments of the present invention are not limited to the specific shapes shown in the drawings but include changes in shape that depend on the fabrication process. Furthermore, it is to be understood that the constituents depicted in the individual drawings might be illustrated as being slightly larger or smaller in consideration of convenience of description.

It will be understood that, although the terms first, second, etc. may be used herein to describe various elements, components, regions, layers and/or sections, these elements, components, regions, layers and/or sections should not be limited by these terms. These terms are only used to distinguish one element, component, region, layer or section from another region, layer or section. Thus, a first element, component, region, layer or section discussed below could be termed a second element, component, region, layer or section without departing from the teachings of the present invention.

Spatially relative terms, such as "beneath", "below", "lower", "above", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if the device in the figures is turned over, elements described as "below" or "beneath" other elements or features would then be oriented "above" the other elements or features. Thus, the exemplary term "below" can encompass both an orientation of above and below. The device may be otherwise oriented (rotated 90 degrees or at other orientations) and the spatially relative descriptors used herein interpreted accordingly.

"Biochip" as used herein refers to a collection of biological components arrayed and attached to a substrate, such as a solid surface. These biological components include, but are not limited to, nucleic acids such as DNA and RNA, oligonucleotides, proteins, peptides, antibodies and antibody fragments, ligands, small molecules, tissue compounds, chemical compounds, and the like, that can interact directly or indirectly with a target sample to, e.g., identify the sample and/or characteristics thereof. Thus, a biochip, as used herein, can be any microarray known to those of skill in the art, *e.g*., DNA microarrays (also referred to as DNA chips or gene chips), RNA microarrays, oligonucleotide microarrays, protein microarrays, peptide microarrays, tissue microarrays, antibody microarrays and/or small molecule microarrays.

### specification.

Spatially relative terms, such as "beneath", "below", "lower", "above", "upper", and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. Like reference numerals refer to like elements throughout the specification.
The present invention will now be described more fully with reference to the accompanying drawings, in which particular embodiments of the invention are shown.

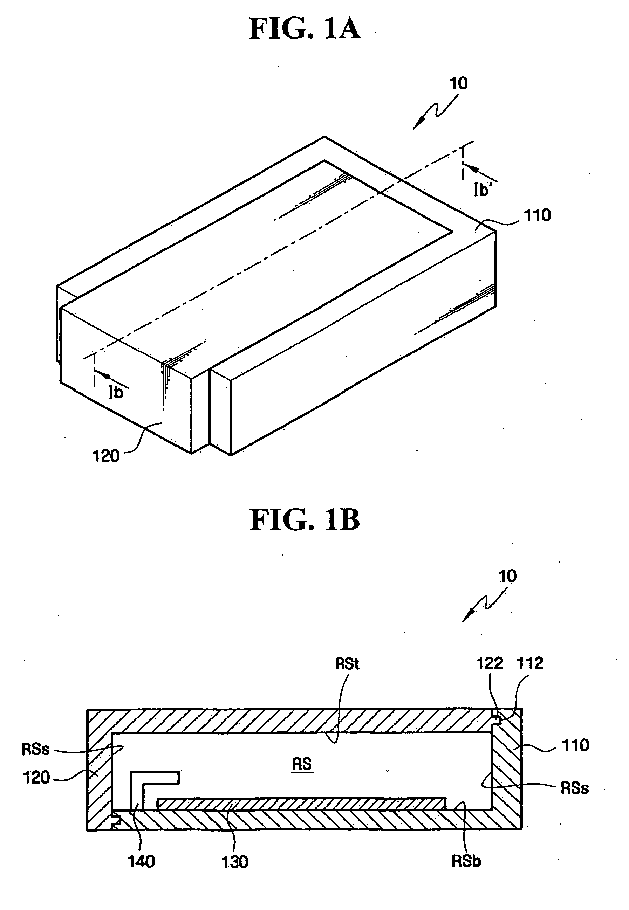

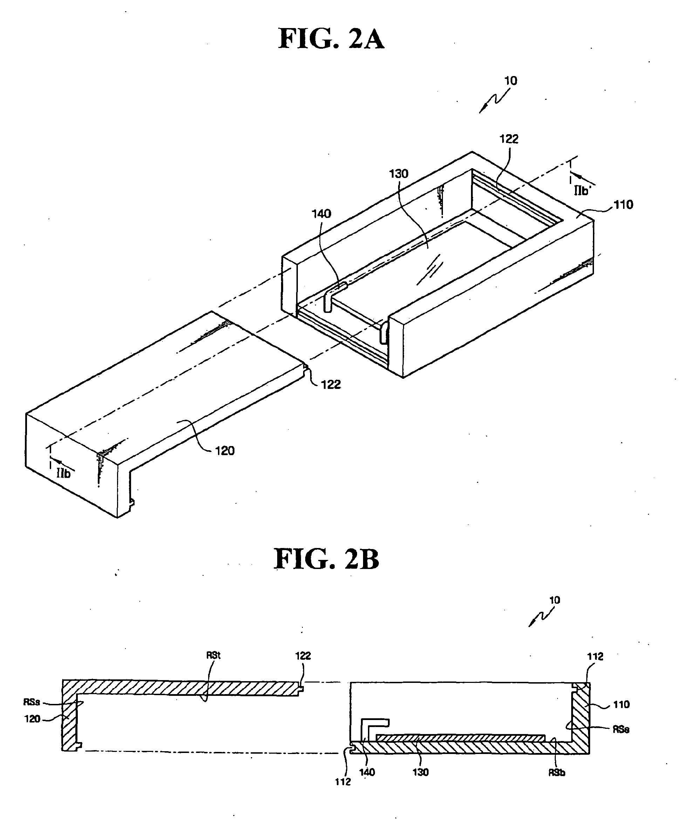

FIG. 1A is a perspective view of a biochip with a first housing and a second housing removably joined, according to an embodiment of the invention. FIG. 1 B is a cross-sectional view of the biochip of FIG. 1A taken along the line Ib'-Ib. FIG. 2A is a perspective view of a biochip with first and second housings separated, according to an embodiment of the invention. FIG. 2B is a cross-sectional view of the biochip of FIG. 2A taken along the line IIb'-IIb.

A biochip kit 10 according to an embodiment of the invention includes a first housing 110 and a second housing 120 that is capable of being removably joined and separated from each other. The first housing 110 includes a biochip disposed in the housing.

The first housing 110 and the second housing 120 can protect the biochip 130 disposed inside. The first housing 110 and the second housing 120 may be, for example, formed of plastic, glass, or metal such as steel or stainless steel. The housings 110 and 120 are usually preferably formed of a rigid material, but is not limited thereto, to provide protection for the biochip from impacts from outside the housings.

A reaction space RS is formed by removably joining the first housing 110 with the second housing 120. The first housing 110 and the second housing 120 each may have a fastening means for joining. The fastening means shown in the figures, by way of example, may include fastening grooves 112 formed on the first housing 110, corresponding to fastening surfaces and fastening prominences 122 formed on the second housing 120. However, in contrast to the example shown, the fastening prominences 122 may be formed on the first housing 110 and the fastening grooves 112 on the second housing 120, and a plurality of grooves and prominences may be formed by combinations thereof. Other than the combination of the fastening grooves 112-to-the fastening prominences 122, key-to-key groove joining, hook joining, or other various joining mechanisms are applicable.

The reaction space RS may be formed by the first and second housings 110 and 120 is a three-dimensional space formed by a top RSt, a bottom RSb, and side RSs structures connected theretoing. Because the conceptional shape of the reaction space RS is defined by the first housing 110 and the inside of the second housing 120, the shape is in accordance to various selective combinations of the geometric shapes thereof. The reaction space RS, that is, may be formed into a rectangular pillar, a square pillar, a circular pillar, an elliptical pillar, etc., depending on the shapes of the first housing 110 and the inside of the second housing 120. The reaction space RS has a rectangular pillar shape herein by way of example for the sake of convenience in description.

Since the reaction space RS is formed by removably joining the first and second housings 110 and 120, the top RSt, bottom RSs, and side RSb structures are originated from at least one of the first and second housings 110 and 120.

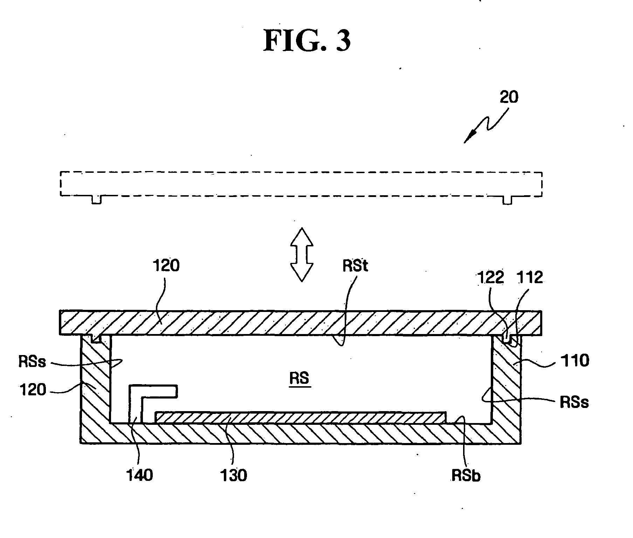

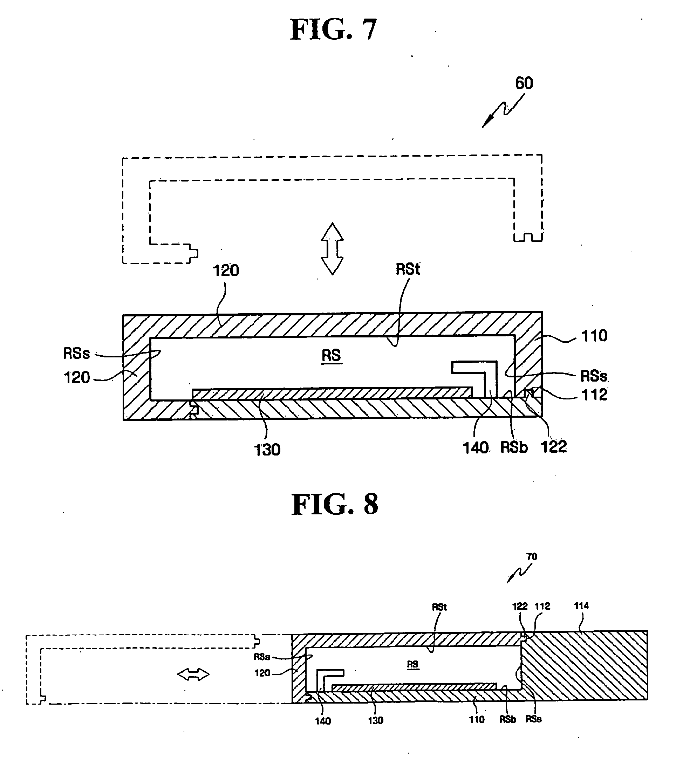

FIGS. 3 to 8 are cross-sectional views of biochips according to other embodiments of the invention. Described in more detail referring to FIGS. 1A to 8, bottom structure RSb of reaction spaces RS of biochip kits 10, 20, 50, and 70 according to some embodiments of the invention may be formed by the first housing 110 (see FIGS. 1B, 2B, 3 to 6, and 8). On the contrary, the bottom structure RSb of the reaction space RS of a biochip kit 60 according to other embodiments of the invention may be formed by the first housing 110 and the second housing 120 (see FIG. 7).

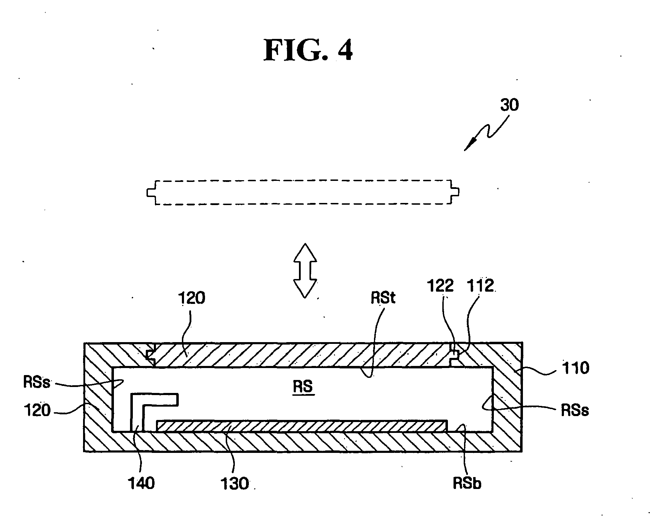

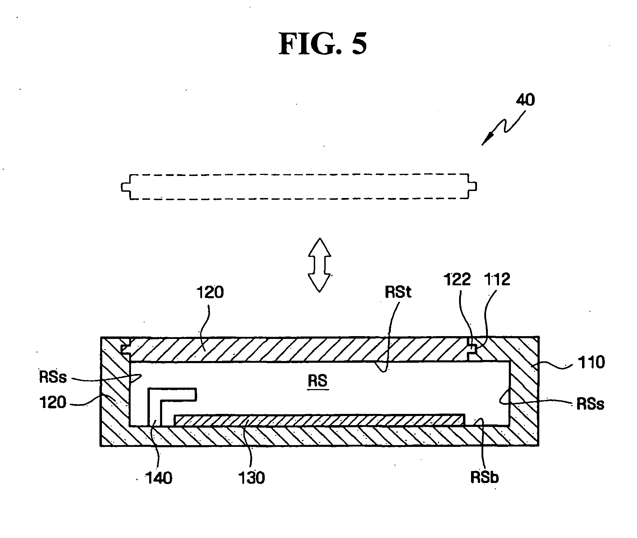

The top structure RSt of the reaction spaces RS of the biochip kits 10, 20, 50, 60, and 70 may be formed by the second housing 120 (see FIGS. 1B, 2B, 3, and 6 to 8). According to some embodiments of the invention, the top structure RSt of the reaction spaces RS of the biochip kits 30 and 40 may be formed by the first housing 110 and the second housing 120 (see FIGS. 4 and 5). However, although the top structure RSt of the reaction space RS may be formed by the first housing 110 and the second housing 120, in particular embodimentsi, the top structure RSt of the reaction space RS formed by the first housing 110 does not cover the top structure RSt of the biochip 130, which will be described later in detail.

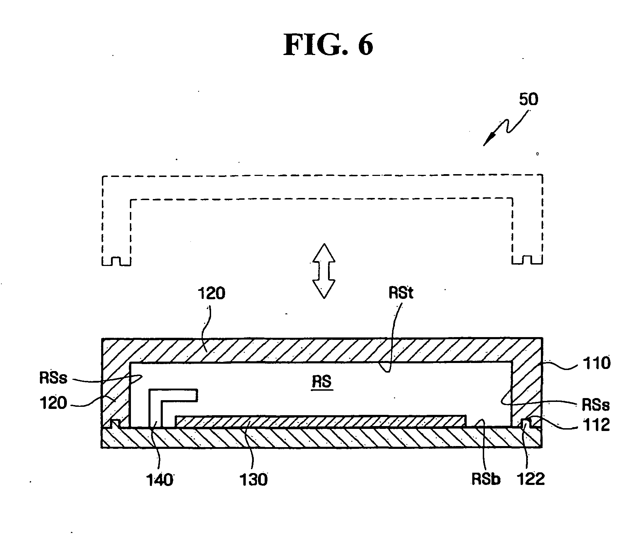

The side structures RSs of the reaction space Rs may be formed only by the first housing 110 (see FIGS. 3 to 5), or only the second housing 120 (see FIGS. 6 and 7), or both first and second housings (see FIGS. 1B, 2B, and 8). In some embodiments of the invention, the first housing 110 forming the side structures RSs of the reaction space RS may have a grip extension 114 extending outside as shown in FIG. 8. The grip extension 114 can provide a margin that allows for gripping the first housing 110, when the first housing 110 and the second housing 120 are joined or separated.

As described above, the first housing 110 may form the bottom structure RSb of the reaction space RS, and the side structures RSs and/or top structure RSt, while the second housing 120 may form the top structure RSt of the reaction space RS, and the side structures RSs and/or top structure RSt. Combinations of the above-mentioned configurations can be variously selected, depending on the size and shape of the biochip 130 that is disposed in the first housing 110, the types of fastening means, joining/separating direction, and the types of detecting devices. Further, it can also be appropriately selected on the basis of the structure of a deck for automatic examination of biochip kits that will be described later.

The biochip kits 10, 20, 30, 40, 50, 60, and 70 according to embodiments described herein may further have inlet/outlet 140 for supplying/discharging fluid, such as a biological sample or cleansing solution, into/out of the reaction space RS. One inlet/outlet 140 is configured to supply and discharge fluid simultaneously. Alternatively, two or more inlet/outlets 140 may be provided and at least one of them may be configured to only supply fluid and at least another one of the inlet/outlets 140 may be configured to only discharge the fluid. The inlet/outlet 140 may be connected with a fluid supplying pipe and/or a fluid discharging pipe located outside the assembly.

The inlet/outlet 140 may be formed at the first housing 110 and/or the second housing 120. For simple description, it will be described in association with the following embodiments and figures wherein the inlet/outlet 140 is formed at the first housing 110, by way of example.

The reaction space RS formed by removably joining the first housing 110 with the second housing 120 may be a substantially closed space. Although coupling reactions of biological samples or other corresponding treatment reactions occur in the biochip kits 10, 20, 30, 40, 50, 60, and 70, the reaction space RS can shield the biological samples against outside pollutants and/or inside reaction conditions can be more readily controlled. The term "'substantially closed space'" includes not only a physically completely closed space, but also a space that is practically spatially closed, but has an opening, such as the inlet/outlet 140, that partially communicates the space with the outside. Even though the inlet/outlet 140 is formed, it may be closed, for example, by a sealing tape (not shown), during a reaction to maintain the cleanliness and/or control the reaction conditions in the reaction space RS.

The substantially closed reaction space RS, formed by removelably joining the first housing 110 with the second housing 120, can isbe released by separating the second housing 120 from the first housing 110 or vice versa.

The biochip 130 is disposed on the first housing 110 and spaced apart from the second housing 120. In particular embodiments, the biochip 130 may be fixed to the bottom of the first housing 110 by an adhesive.

When the first housing 110 is removably joined to the second housing 120, the upper surface of the biochip 130 is covered by the second housing 120, while the first housing does not cover the biochip 130. As a result, when the second housing 120 is separated from the first housing 110, the reaction space RS is released and the upper surface of the biochip 130 may be correspondingly exposed. Accordingly, an examining device for the biochip 130 can approach the upper surface of the biochip 130 with close proximity, allowing more precise examination.

The biochip 130, for example, may be applicable to gene expression analysis/profile, genotyping or comparative genomic hybridization, detection of mutation/polymorphism, such as a single nucleotide polymorphism (SNP), protein/peptide analysis reaction patterns, screening potential medicines, and synthesizing/developing /makingnew medicines.

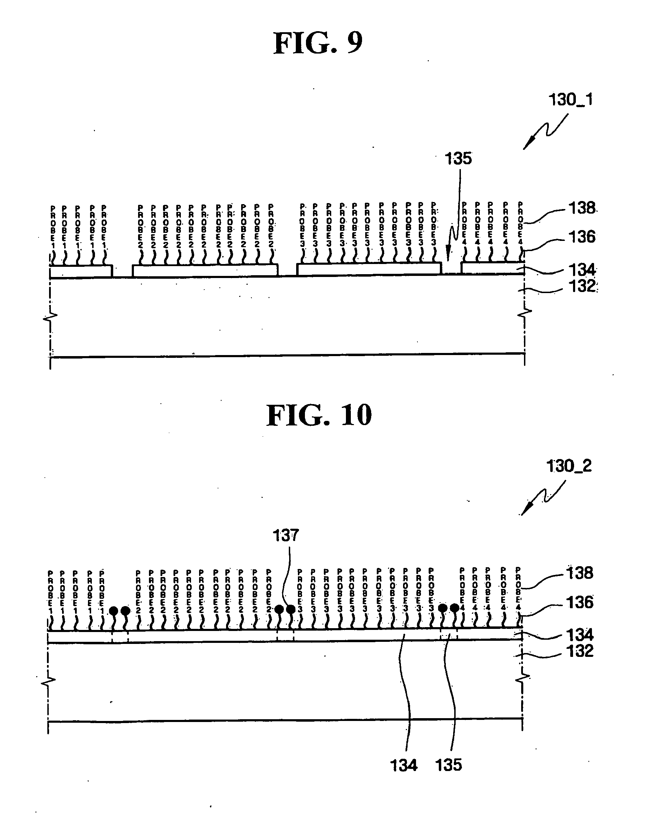

FIGS. 9 to 13 show examples of an applicable biochip in detail.

FIGS. 9 to 13 are cross-sectional views of biochips included in the biochip kits according to some embodiments of the invention. Referring to FIGS. 9 to 13, biochips 130_1, 130_2, 130_3, 130_4, and 130_5 individually include a substrate 132 and an active region 134 that is disposed on the substrate 132 to fix a plurality of probes thereon.

The substrate 132 may be flexible or rigid. An applicable flexible substrate may include ofa nylon or nitrocellulose membrane, or a plastic film. A rigid substrate may be a semiconductor substrate such as silicon, or a transparent glass substrate formed, for example, of soda lime glass. For the biochip kits according to some embodiments of the invention, the biochips 130_1, 130_2, 130_3, 130_4, and 130_5 can be directly examined through the upper surfaces, thereby facilitating examination of biochips 130_1, 130_2, 130_3, 130_4, and 130_5 even though the substrate 132 is formed of a semiconductor substrate, or an impervious substrate such as a membrane or plastic film as described above.

In particular, a semiconductor substrate may have an advantage for which manufacturing processes and photolithography processes for a variety of thin films are directly applicable, at least because the manufacturing process of semiconductor elements has already been established.

The active region 134 may be formed on the substrate 132. In particular embodiments, the active region 134 is formed of a substance that is substantially stable and generally not hydrolyzed in hybridization analysis, for example, in contact with phosphate at pH about 6 to about 9 or TRIS buffer. For example, the active region 134 may be formed of a silicon oxide film such as PE-TEOS film, HDP oxide film, P-SiH₄ oxide film, or thermal oxide film, a silicate such as hafnium silicate or zirconium silicate, a metal oxynitride film such as silicon oxynitride film, hafnium oxynitride film, or zirconium oxynitride film, a metal oxide film such as titanium oxide film, tantalum oxide film, aluminum oxide film, hafnium oxide film, zirconium oxide film, or ITO, a metal such as polyamide, polyamine, gold, silver, copper, or palladium, or a polymer such as polystyrene, polyacrylic acid, or polyvinyl.

The active region 134 may be divided into numerous pieces by probe cell separating regions 135. The active region 134 may be distinguished from the probe cell separating regions on the basis of whether probes 138 are fixed thereon them. As used herein, a "probe cell" refers to a defined area of the microarray that includes a functional group that may act as a linker or is formed of a material that provides a functional group that can act as a linker once the material is treated by a surface treatment such as ozone, acid and/or base. Each probe cell may be isolated from the other probe cells in the microarray by a probe cell separation area. The term "probe," as used herein, refers to a molecule attached to the probe cell. Examples of a probe include a molecular probe formed of DNA, RNA, oligonucleotides, proteins, peptides, antibodies and antibody fragments, ligands, small molecules, tissue compounds, chemical compounds, and the like, that can interact directly or indirectly with a target sample to, e.g., identify the sample and/or characteristics thereof.

Several probes 138 may be coupled to the active region 134. In detail, the number of probes 138 that are coupled to each active region 134 may be the same, but different types of probes 138 may be coupled to the separated active regions 134. The probes 138 can vary according to biological samples to be tested. For example, the probe 138 may be an oligonucleotide probe.

The coupling between the active region 134 and the probe 138 may be provided through a linker 136 therebetween. As used herein, "coupling" or "coupled," is meant to signify the connection, linking, conjugation and/or attachment of the groups or molecules coupled. Such coupling may occur, e.g., via covalent and/or non-covalent interactions.

As for the biochip 130_1 according to some embodiments of the invention, as shown in FIG. 9, by way of example, the active region 134 may be patterned on the substrate 132. In FIG. 9, the regions on the substrate with the active region 134 removed represent the probe cell separating regions 135.

As for the biochip 130_2 according to some embodiments of the invention, as shown in FIG. 10, by way of example, the active region 134 may not be physically patterned, but instead, divided by probe cell separating regions 135 where only inactive linkers 137 may be coupled at predetermined regions, without the probes 138.

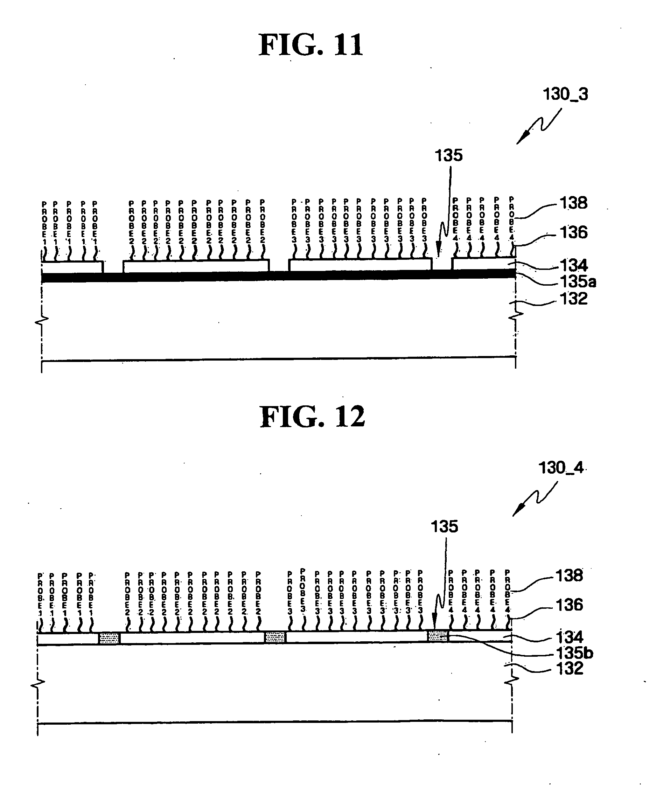

As for the biochip 130_3 according to some embodiments of the invention, as shown in FIG. 11, by way of example, a coupling blocking film 135a may be formed on the substrate 132 and an active region 134 may be physically patterned on the coupling blocking film 135a. The coupling blocking film 135a may be, for example, formed of fluoride containing a fluorine group as in a fluorosilane film. In FIG. 11, the probe cell separating regions 135 represent the upper surface of the coupling blocking film 135a while the active region 134 is removed.

As for the biochip 130_4 according to some embodiments of the invention, as shown in FIG. 12, by way of example, the active region 134 may be patterned as in FIG. 9 and a blocking filler 135b may be filled in the region while the active region 134 may be removed. The blocking filler 135b may be formed of a film having good characteristics in coupling blocking and/or gap filling, for example, fluorosilane or polysilicon. In FIG. 12, the probe cell separating regions 135 may be the upper surface of the blocking filler 135b.

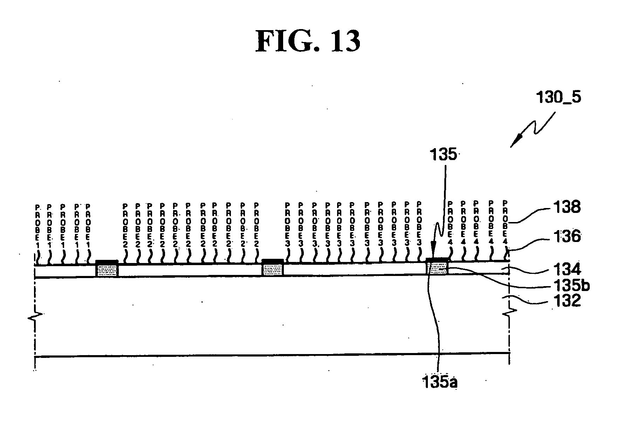

As for the biochip 130_5 according to some embodiments of the invention, as shown in FIG. 13 by way of example, the active region 134 may have at least a substantially similar the same configuration, if not the same, as in FIG. 12, but the coupling blocking film 135a may be formed on the blocking fillers 135b filled in the regions while the active region 134 may be removed. Therefore, in FIG. 13, the probe cell separating region 135 may be the upper surface of the coupling blocking film 135a.

The biochips 130_1, 130_2, 130_3, 130_4, and 130_5 shown in FIGS. 9 to 13, by way of examples, may replace the biochips 130 included in the biochip kits 10, 20, 30, 40, 50, 60, and 70 of FIGS. 1A to 8.

A method of testing a biological sample using the biochip kits as described above will be described hereafter. As a measure of convenience, a method of testing a biological sample is described in the following embodiment, using the biochip kit shown in FIG. 8, by way of example,; however, it should be understood that the method that is described in association with the present invention is applicable to the biochips according to the other embodiments of the present invention.

First, as shown in a solid line in FIG. 8, a substantially closed reaction space RS may be defined by joining the first housing 110 with the second housing 120 of the biochip kit 70.

Subsequently, a biological sample may be provided into the reaction space RS. When the biochip kit 70 has the inlet/outlet 140, the biological sample may be provided through the inlet/outlet 140. If the biochip kit 70 does include the inlet/outlet 140, the biological sample may be supplied before the first housing 110 is joined with the second housing 120, i.e., while the second housing 120 is separated. The biological sample may include, for example, a single- stranded ined DNA (sDNA). A fluorescent substance may be attached to the sDNA.

Thereafter, a coupling reaction of the probes 138 and the biological sample may occur by controlling reaction conditions. When the probes 138 are oligonucleotide probes and the biological sample includes sDNA, the coupling reaction may be a hybridization reaction. When the biochip kit 70 includes the inlet/outlet 140, the inlet/outlet may be closed by a sealing tape (not shown) to completely close the reaction space during the coupling reaction.

The biological sample may include sDNA; however, when a fluorescent substance is not attached to the DNA, the method may further include providing a fluorescent stain or dye inside the reaction space RS and attaching the stain or dye to the probes 130 that have undergone the coupling reaction, for example, a hybridization reaction.

After the coupling reaction, the biological sample may be discharged from the reaction space RS. The biological sample may be discharged through the inlet/outlet 140.

After the biological sample is discharged, optionally, a cleansing solution may be supplied into the reaction space RS to remove the remaining biological sample inside the reaction space RS.

Further, dry gas, may be optionally supplied into the reaction space RS to dry the upper surface of the biochip 130. Nitrogen gas may be used as the dry gas and can be introduced into or discharged from the reaction space RS through the inlet/outlet 140, for example.

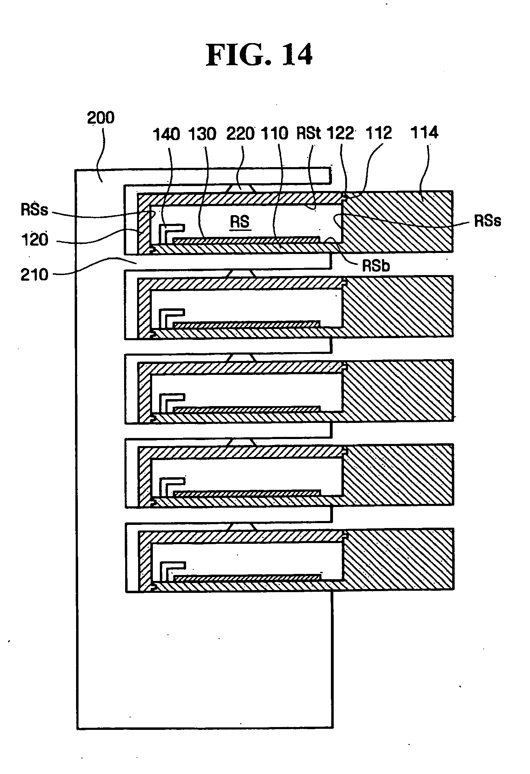

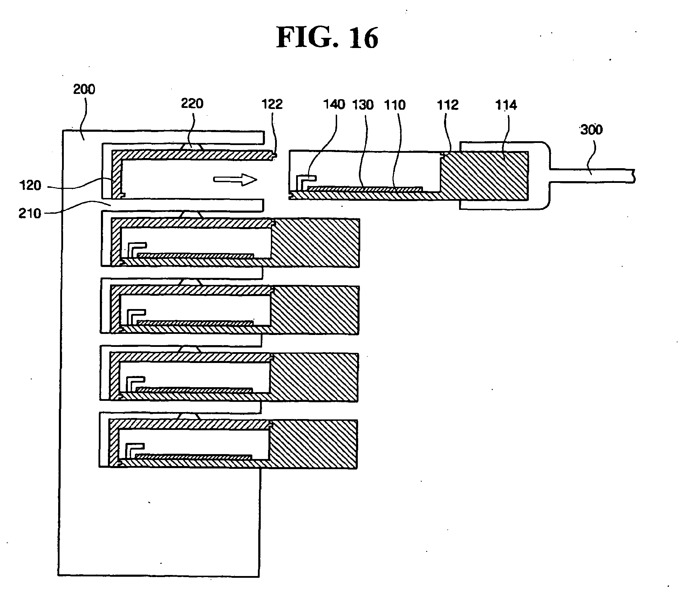

Thereafter, as shown in a dotted line in FIG. 8, the second housing 120 may be separated from the first housing 110 of the biochip kit 70. As the second housing 120 is separated, the upper surface of the biochip 130 may be exposed to the outside at least because the first housing 110 no longer covers the upper surface. A detector may be situated close to the exposed biochip 130 to detect whether the probes 138 are bound to the biological sample. In particular embodiments, the whether the biochip is examined by using an automatic system in order to determine whether the biochip is exposed and bound. A method of testing whether the biochip is exposed and bound by using an automatic system is now described in detail with reference to FIGS. 14 to 17.

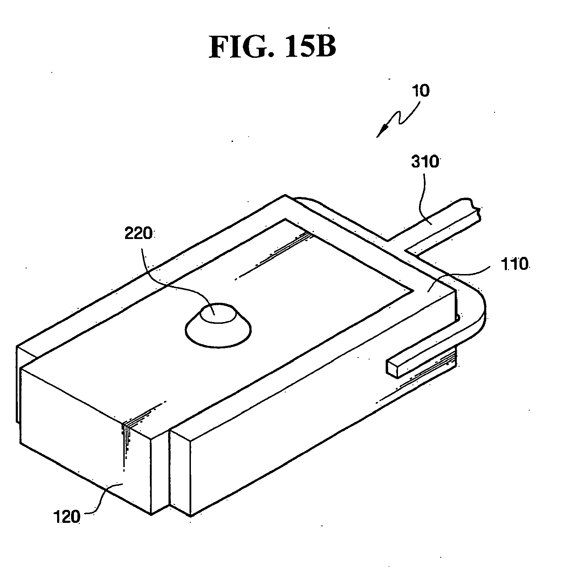

FIGS. 14, 15A, 16, and 17 are cross-sectional views illustrating a method of testing a biological sample according to embodiments of the invention. FIG. 15B is a perspective view illustrating a manner of gripping the first housing of a biochip kit according to some embodiments.

Referring to FIG. 14, the biochip kit 70 having undergone a coupling reaction may be loaded into a loading section 210 of an automatic deck 200. The automatic deck 200 has a plurality of loading sections 210 to load each biochip kit 70 therein. Therefore, several biochip kits 70 can be simultaneously loaded in the loading sections 210 of the automatic deck 200, respectively.

The automatic deck 200 may further include grips 220 to grip the second housings 120 of the biochip kits 70. At least one grip 220 may be provided for each loading section 210. The grip 220, for example, may have a vacuum absorber that vacuum-adsorbs the second housing 120 from the top. The second housings 120 of the biochip kits 70 are respectively gripped to the automatic deck 200 by actuating the grip 220.

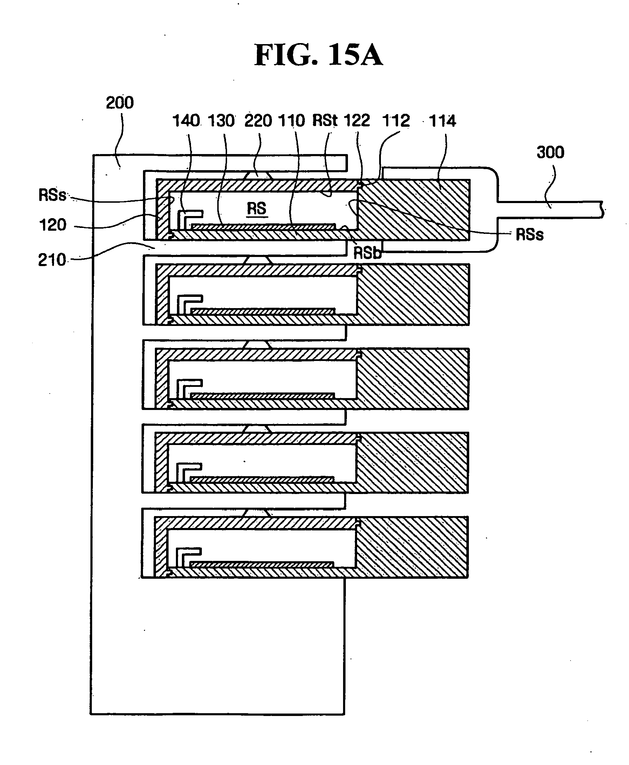

Referring to FIG. 15A, a conveying chuck 300 may be actuated to the automatic deck 200 and grips the first housing 110 of the biochip kit 70 loaded in the first loading section 210. As shown in FIG. 15A, when the first housing 110 of the biochip kit 70 has the grip extension 114, the conveying chuck vertically grips the grip extension 114. If the first housings 110 of the biochip kits 10, 20, 30, 40, 50, 60, and 70 according to the embodiments shown in FIGS. 1A to 7 each do not have a grip extension, the conveying chuck 300 may horizontally grip the sides of the first housings 110 as shown in FIG. 15B, by way of example.

After gripping the first housing 110, as the conveying chuck 300 moves, the fastening by the fastening groove 112 of the first housing 110 and the fastening prominence 122 of the second housing 120 may be released and the second housing 120 may be separated from the first housing 110.

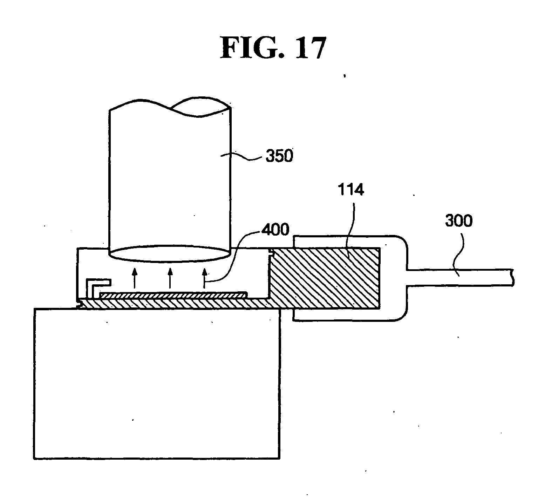

Referring to FIG. 17, the chuck 300 with the first housing 110 gripped moves to the detector 350. For example, the detector 350 closely approaches the upper surface of the biochip 130 exposed to the outside in the first housing 110 and tests the coupling of the probes 138 and a biological sample.

For example, if the biological sample has a fluorescent substance or is provided with a fluorescent stain or dye, when light with a first wavelength is radiated onto the upper surface of the biochip 130, a coupling reaction occurs, such as a hybridization reaction. When a fluorescent substance remains in the biochip 130 during the coupling reaction, the light having a first wavelength results in the emission of light from the fluorescent substance and another light 400 having a second wavelength different from the first wavelength may be emitted. When a coupling reaction does not occur and the fluorescent substance does not remain, light having another wavelength different from the first wavelength is not emitted. Accordingly, it is possible to test whether the coupling reaction occurs, by detecting the light 400 having the second wavelength. The detector 350 may include a light detecting means, such as CCD (Charge Coupled Device) or CIS (CMOS Image Sensor) to detect light having a second wavelength.

CCD or CIS may also be used to analyze the amount and wavelength of emitted light by concentrating the light using a lens and converting it into an electrical signal. Unlike the example, provided for illustrative purposes in FIG. 17, a plurality of lenses of CCD or CIS may be disposed at each active region 134 of the biochip 130.

The light 400 having a second wavelength that is emitted from the fluorescent substance has basically a random optical path. Therefore, in some embodiments, the detector 350, i.e. the lens of CCD or CIS in the detector 350 or the associated objective lens, is disposed as close as possible to a light emitting source in order for light to be concentrated at a greater amount. In particular, in general, the more the design rule decreases, the necessity for a closer approach increases. Further, when another medium is provided between the light emitting source and the lens, the optical path may be changed by Snell's law and, particularly, when the interface of the medium is rough, the optical path may become more irregular. In addition, because light generally changes in wavelength when it travels through different mediums, it can be problematic to measure an accurate wavelength.

However, as for the biochip kit 70 according to the embodiments of the present invention, the upper surface of the biochip 130 may be exposed, and in some embodiments, completely exposed, to the outside by separating the second housing 120 from the first housing 110. Accordingly, CCD or CIS or the associated objective lens can approach the biochip 130 directly without any medium except for air. Therefore, because light can be sufficiently concentrated and the accurate wavelength can be detected as well, reliability can be improved in testing. Although the upper surface of the biochip 130 is exposed, because the coupling reaction is completed during the testing, contamination does not present a significant problem. Further, when the conveying path of the automatic deck 200, the conveying chuck 300 and the detecting place of the detector 350 are efficiently controlled in one device, the biochip 130, for practical purposes, is not contaminated.

After the test for the biochip kit 70 loaded in the first loading section 210 of the automatic deck 200 is completed as described above, the test is sequentially repeated for the next biochip kits 70.

According to the methods of testing a biological sample according to some of an embodiments of the invention as described above, several biochip kits 70 can be automatically tested using only the automatic deck 200 and the conveying chuck 300, having a simple configuration. As a result, test efficiency can be improved.

## Claims

1. A method of testing a biological sample, comprising:
providing a kit (10) comprising a first housing (110), a biochip (130) disposed inside the first housing (110) and configured to comprise a plurality of molecular probes, and a second housing (120) joined to the first housing (110) and configured to form a reaction space (RS) with the first housing (110) and to cover the biochip (130), wherein the reaction space (RS) comprises a top region (RSt), a bottom region (RSb), and side regions (RSs) that spatially connect the top region (RSt) and the bottom region (RSb), and wherein the first housing (110) and the second housing (120) are removably joined;
conducting a coupling reaction of the biological sample with the molecular probes (138) by introducing the biological sample to the reaction space (RS);
separating the first and second housings (110, 120) to expose an upper surface of the biochip (130); and
testing the binding of the molecular probes (138) to the biological sample by placing a detector (350) in close proximity to the biochip (130) to allow detection of said binding,
wherein the side regions (RSs) of the reaction space (RS) are formed by the joined first and second housings (110, 120) and
wherein testing the binding of the molecular probes to the biological sample is performed after separating the first and second housings (110, 120) such that only air medium is disposed between the detector (350) and the exposed biochip (130) during the testing,
**characterized in that** in the separating step, the second housing (120) is gripped by a grip (220) from the top, and the first housing (110) is gripped and conveyed by a conveying chuck (300) to separate the first housing (110) from the second housing (120), wherein fastening surfaces and fastening prominences (122) formed on the first or the second housing (110, 120) are removed from corresponding fastening grooves formed on the other one of the first and the second housing (110, 120) when the conveying chuck conveys the first housing (110).

2. The method of claim 1, wherein:
(a) the biological sample comprises a fluorescent substance; and
(b) testing the binding of the molecular probes (138) to the biological sample comprises detecting light emitted from the fluorescent substance.

3. The method of claim 2, wherein the detector (350) comprises a Charge Coupled Device (CCD) or a CMOS Image Sensor (CIS).

4. The method of claim 1, wherein the molecular probes (138) are oligonucleotide probes, and the coupling reaction is a hybridization reaction.

5. The method of any one of claims 1 to 4, further comprising:
loading a plurality of biochip kits (10) into an automatic deck (200), wherein each biochip kit comprises (i) a first housing (110), (ii) a biochip (130) disposed inside the first housing (110) and having a plurality of molecular probes (138) that have undergone a coupling reaction, and (iii) a second housing (120) joined to the first housing (110) configured to form a reaction space (RS) and to cover the biochip (130);
actuating the automatic deck (200) to grip the second housing (120) of each of the biochip kits (10) by a grip (220) from the top;
actuating a conveying chuck (300) to move toward the automatic deck (200) and grip the first housing (110) of an n^{th} biochip kit (10), wherein n is an integer of 1 or more;
separating the first and second housings (110, 120) of the n^{th} biochip kit (10) gripped by the automatic deck (200) by actuating the conveying chuck (300) gripping the first housing (110) of the n^{th} biochip kit (10) to move in a horizontal direction; and
testing the biochip (130) disposed inside the first housing (110) of the n^{th} biochip kit (10) such that only air medium is disposed between the detector (350) and the biochip (130) disposed inside the first housing (110) of the n^{th} biochip kit (10) by conveying the first housing (110) of the separated n^{th} biochip kit (10) to a detector (350).

6. The method of claim 5, wherein:
the first housing (110) of the biochip kits (10) each comprises a grip extension (114) configured to extend outside the reaction spaces (RS); and
the conveying chuck (300) grips the first housing (110) of the n^{th} biochip kit (10) by gripping the grip extension (114).

7. The method of claim 5, wherein after testing the biochip (130) disposed inside the first housing (110) of the n^{th} biochip kit (10), the method further comprising:
actuating the conveying chuck (300) to move toward the automatic deck (200) and grip the first housing (110) of an n^{th+1} biochip kit (10);
separating the first housing (110) from the second housing (120) of the n^{th}+1 biochip kit (10) gripped by the automatic deck (200) by actuating the conveying chuck (300) gripping the first housing (110) of the n^{th}+1 biochip kit (10); and
testing the biochip kit (130) disposed inside the first housing (110) of the n^{th}+1 biochip kit (10) such that only air medium is disposed between the detector (350) and the biochip (130) disposed inside the first housing (110) of the n^{th} biochip kit (10) is disposed by conveying the first housing (110) of the separated n^{th}+1 biochip kit (10) to a detector (350).

8. The method of claim 1, wherein the first housing (110) is conveyed in the horizontal direction by the conveying chuck (300).

## Patentansprüche

1. Verfahren zum Testen einer biologischen Probe, aufweisend:
Bereitstellen eines Sets (10), bestehend aus einem ersten Gehäuse (110), einem Biochip (130), der sich im Innern des ersten Gehäuses befindet (110) und der so konfiguriert ist, dass er eine Vielzahl molekularer Sonden aufnimmt, sowie einem zweiten Gehäuse (120), das mit dem ersten Gehäuse (110) verbunden und so konfiguriert ist, dass es mit dem ersten Gehäuse (110) einen Reaktionsraum (RS) bildet und den Biochip (130) überdeckt, wobei der Reaktionsraum (RS) aus einem oberen Bereich (RSt), einem unteren Bereich (RSb) sowie seitlichen Bereichen (RSs) besteht, die den oberen Bereich (RSt) und den unteren Bereich (RSb) räumlich miteinander verbinden, und wobei das erste Gehäuse (110) und das zweite Gehäuse (120) abnehmbar miteinander verbunden sind;
Durchführen einer Kupplungsreaktion der biologischen Probe mit den molekularen Sonden (138) durch Einbringen der biologischen Probe in den Reaktionsraum (RS);
Trennen des ersten Gehäuses vom zweiten (110, 120) zum Freilegen der oberen Oberfläche des Biochips (130); und
Testen der Bindung der molekularen Sonden (138) an die biologische Probe, indem ein Detektor (350) in großer Nähe zum Biochip (130) platziert wird, um die genannte Bindung zu ermitteln,
wobei die Seitenbereiche (RSs) des Reaktionsraums (RS) aus den zusammengefügten ersten und zweiten Gehäusen (110, 120) bestehen und
wobei das Testen der Bindung der molekularen Sonden an die biologische Probe nach der Trennung des ersten und zweiten Gehäuses (110, 120) so erfolgt, dass sich zwischen dem Detektor (350) und dem freigelegten Biochip (130) während des Testens nur Luft als Medium befindet,
**dadurch gekennzeichnet, dass** während des Trennvorgangs das zweite Gehäuse (120) von einem Griff (220) von oben gegriffen wird und das erste Gehäuse (110) von einer Transporthaltevorrichtung (300) gegriffen und befördert wird, um das erste Gehäuse (110) von dem zweiten Gehäuse (120) zu trennen, wobei Befestigungsflächen und Befestigungsvorsprünge (122), die auf dem ersten oder dem zweiten Gehäuse (110, 120) ausgebildet sind, aus den passenden Befestigungskerben, die auf dem jeweils anderen erstem oder zweiten Gehäuse (110, 120) ausgebildet sind, entfernt werden, wenn die Transporthaltevorrichtung das erste Gehäuse (110) befördert.

2. Verfahren nach Anspruch 1, wobei:
(a) die biologische Probe eine fluoreszierende Substanz enthält; und
(b) das Testen der Bindung der molekularen Sonden (138) an die biologische Probe die Erfassung von Licht, das von der fluoreszierenden Substanz abgestrahlt wird, beinhaltet.

3. Verfahren nach Anspruch 2, wobei der Detektor (350) einen Charge-Coupled-Device- (CCD-) oder CMOS-Bildsensor (CIS) aufweist.

4. Verfahren nach Anspruch 1, wobei es sich bei den molekularen Sonden (138) um Oligonukleotid-Sonden handelt und es sich bei der Kupplungsreaktion um eine Hybridisierungsreaktion handelt.

5. Verfahren nach einem der Ansprüche 1 bis 4, weiter aufweisend:
Laden einer Vielzahl von Biochip-Sets (10) in ein automatisches Deck (200), wobei jedes Biochip-Set (i) ein erstes Gehäuse (110), (ii) einen Biochip (130), der sich im Innern des ersten Gehäuses (110) befindet und über eine Vielzahl von molekularen Sonden (138) verfügt, die eine Kupplungsreaktion durchlaufen haben, und (iii) ein zweites Gehäuse (120), das mit dem ersten Gehäuse (110) verbunden und so konfiguriert ist, dass es mit dem ersten Gehäuse einen Reaktionsraum (RS) bildet und den Biochip überdeckt (130), aufweist;
Betätigen des automatischen Decks (200) derart, dass das zweite Gehäuse (120) jedes der Biochip-Sets mit einem Griff (220) von oben gegriffen wird;
Betätigen einer Transporthaltevorrichtung (300), die sich zum automatischen Deck (200) bewegt und das erste Gehäuse (110) eines n-ten Biochip-Sets greift, wobei es sich bei n um eine ganze Zahl größer als oder gleich 1 handelt;
Trennen des ersten und zweiten Gehäuses (110, 120) des vom automatischen Deck (200) gegriffenen n-ten Biochip-Sets (10) durch Betätigen der Transporthaltevorrichtung (300), die das erste Gehäuse (110) des n-ten Biochip-Sets (10) greift, damit sich diese in eine horizontale Richtung bewegt; und
Testen des Biochips (130), der sich im ersten Gehäuse (110) des n-ten Biochip-Sets (10) befindet, so dass sich nur Luft als Medium zwischen dem Detektor (350) und dem Biochip (130) befindet, der sich im ersten Gehäuse (110) des n-ten Biochip-Sets (10) befindet, indem das erste Gehäuse (110) des getrennten n-ten Biochip-Sets (10) zu einem Detektor (350) befördert wird.

6. Verfahren nach Anspruch 5, wobei:
das erste Gehäuse (110) der Biochip-Sets (10) jeweils eine Griffverlängerung (114) aufweist, die so konfiguriert ist, dass sie sich außerhalb des Reaktionsraums (RS) erstreckt; und
die Transporthaltevorrichtung (300) das erste Gehäuse (110) des n-ten Biochip-Sets (10) durch Greifen der Griffverlängerung (114) greift.

7. Verfahren nach Anspruch 5, wobei das Verfahren nach dem Testen des im ersten Gehäuse (110) des n-ten Biochip-Sets (10) angebrachten Biochips (130) weiter aufweist:
Betätigen der Transporthaltevorrichtung (300), die sich zum automatischen Deck (200) bewegt und das erste Gehäuse (110) eines (n+1)-ten Biochip-Sets (10) greift;
Trennen des ersten (110) von dem zweiten Gehäuse (120) des vom automatischen Deck (200) gegriffenen (n+1)-ten Biochip-Sets (10) durch Betätigen der Transporthaltevorrichtung (300), die das erste Gehäuse (110) des (n+1)-ten Biochip-Sets (10) greift; und
Testen des Biochip-Sets (130), der sich im ersten Gehäuse (110) des (n+1)-ten Biochip-Sets (10) befindet, so dass sich nur Luft als Medium zwischen dem Detektor (350) und dem Biochip (130) befindet, der sich im ersten Gehäuse (110) des n-ten Biochip-Sets (10) befindet, indem das erste Gehäuse (110) des getrennten (n+1)-ten Biochip-Sets (10) zu einem Detektor (350) befördert wird.

8. Verfahren nach Anspruch 1, wobei das erste Gehäuse (110) von der Transporthaltevorrichtung (300) in horizontaler Richtung befördert wird.

## Revendications

1. Procédé d'analyse d'un échantillon biologique, comprenant :
la fourniture d'un kit (10) comprenant un premier logement (110), une biopuce (130) disposée à l'intérieur du premier logement (110) et configurée pour comprendre une pluralité de sondes moléculaires, et un second logement (120) joint au premier logement (110) et configuré pour former un espace réactionnel (RS) avec le premier logement (110) et pour couvrir la biopuce (130), où l'espace réactionnel (RS) comprend une région supérieure (RSt), une région inférieure (RSb), et des régions latérales (RSs) qui relient spatialement la région supérieure (RSt) et la région inférieure (RSb), et où le premier logement (110) et le second logement (120) sont joints de manière amovible ;
la conduction d'une réaction de couplage de l'échantillon biologique avec les sondes moléculaires (138) par l'introduction de l'échantillon biologique dans l'espace réactionnel (RS) ;
la séparation des premier et second logements (110, 120) pour exposer une surface supérieure de la biopuce (130) ; et
l'analyse de la liaison des sondes moléculaires (138) à l'échantillon biologique en plaçant un détecteur (350) en étroite proximité avec la biopuce (130) pour permettre la détection de ladite liaison,
où les régions latérales (RSs) de l'espace réactionnel (RS) sont formées par les premier et second logements joints (110, 120) et
où l'analyse de la liaison des sondes moléculaires à l'échantillon biologique est réalisée après séparation des premier et second logements (110, 120) de telle manière que seul le milieu de l'air est disposé entre le détecteur (350) et la biopuce exposée (130) durant l'analyse,
**caractérisé en ce que** dans l'étape de séparation, le second logement (120) est agrippé par une pince (220) depuis le haut, et le premier logement (110) est agrippé et transporté par une plaque de transport (300) pour séparer le premier logement (110) du second logement (120), où les surfaces de fixation et les protubérances de fixation (122) formées sur le premier ou le second logement (110, 120) sont retirées des rainures de fixation correspondantes sur l'autre des premier et second logements (110, 120) lorsque la plaque de transport transporte le premier logement (110).

2. Procédé selon la revendication 1, dans lequel :
(a) l'échantillon biologique comprend une substance fluorescente ; et
(b) l'analyse de la liaison des sondes moléculaires (138) à l'échantillon biologique comprend la détection de la lumière émise à partir de la substance fluorescente.

3. Procédé selon la revendication 2, dans lequel le détecteur (350) comprend un dispositif à couplage de charge (CCD) ou un capteur d'images CMOS (CIS).

4. Procédé selon la revendication 1, dans lequel les sondes moléculaires (138) sont des sondes oligonucléotidiques, et la réaction de couplage est une réaction d'hybridation.

5. Procédé selon l'une quelconque des revendications 1 à 4, comprenant en outre :
le chargement d'une pluralité de kits de biopuces (10) dans une plate-forme automatique (200), où chaque kit de biopuce comprend (i) un premier logement (110), (ii) une biopuce (130) disposée à l'intérieur du premier logement (110) et comportant une pluralité de sondes moléculaires (138) qui ont subi une réaction de couplage, et (iii) un second logement (120), joint au premier logement (110) configuré pour former un espace réactionnel (RS) et pour couvrir la biopuce (130) ;
l'actionnement de la plate-forme automatique (200) pour agripper le second logement (120) de chacun des kits de biopuces (10) par une pince (220) depuis le haut ;
l'actionnement d'une plaque de transport (300) de manière à ce qu'elle se déplace vers la plate-forme automatique (200) et agrippe le premier logement (110) d'un n^{ème} kit de biopuce (10), où n est un nombre entier de 1 ou plus ;
la séparation des premier et second logements (110, 120) du n^{ème} kit de biopuce (10) agrippés par la plate-forme automatique (200) par l'actionnement de la plaque de transport (300) agrippant le premier logement (110) du n^{ème} kit de biopuce (10) de manière à ce qu'il se déplace dans une direction horizontale ; et
l'analyse de la biopuce (130) disposée à l'intérieur du premier logement (110) du n^{ème} kit de biopuce (10) de telle manière que seul le milieu de l'air est disposé entre le détecteur (350) et la biopuce (130) disposée à l'intérieur du premier logement (110) du n^{ème} kit de biopuce (10) par transport du premier logement (110) du n^{ème} kit de biopuce (10) séparé vers un détecteur (350).

6. Procédé selon la revendication 5, dans lequel :
le premier logement (110) des kits des biopuces (10) comprend chacun une extension de pince (114) configurée pour s'étendre en dehors des espaces réactionnels (RS) ; et
la plaque de transport (300) agrippe le premier logement (110) du n^{ème} kit de biopuce (10) par agrippement de l'extension de pince (114).

7. Procédé selon la revendication 5, dans lequel après l'analyse de la biopuce (130) disposée à l'intérieur du premier logement (110) du n^{ème} kit de biopuce (10), le procédé comprenant en outre :
l'actionnement de la plaque de transport (300) de manière à ce qu'elle se déplace vers la plate-forme automatique (200) et agrippe le premier logement (110) du n^{ème}+1 kit de biopuce (10) ;
la séparation du premier logement (110) du second logement (120) du n^{ème}+1 kit de biopuce (10) agrippé par la plate-forme automatique (200) par actionnement de la plaque de transport (300) agrippant le premier logement (110) du n^{ème}+1 kit de biopuce (10) ; et
l'analyse du kit de biopuce (130) disposé à l'intérieur du premier logement (110) du n^{ème}+1 kit de biopuce (10) de telle manière que seul le milieu de l'air est disposé entre le détecteur (350) et la biopuce (130) disposée à l'intérieur du premier logement (110) du n^{ème} kit de biopuce (10) par transport du premier logement (110) du n^{ème}+1 kit de biopuce (10) vers un détecteur (350).

8. Procédé selon la revendication 1, dans lequel le premier logement (110) est transporté dans la direction horizontale par la plaque de transport (300).
